# EUROPEAN PATENT APPLICATION

(11) **EP 4 129 646 A1**
(43) Date of publication of application: **08.02.2023**
(21) Application number: 21775783.0
(22) Date of filing: 26.03.2021
(51) Int. Cl.: B32B 9/00, B32B 9/04, B32B 27/00, B32B 27/06, B32B 15/01, B32B 15/04, B32B 15/08, B32B 15/20, C09J 7/38, A01P 3/00, A01N 25/34, A01N 59/20

(54) **ANTIBACTERIAL MEMBER**

(30) Priority: 27.03.2020 JP 2020057131; 25.06.2020 JP 2020109482; 07.07.2020 JP 2020116807
(71) Applicant: Mitsubishi Materials Corporation, Tokyo 100-8117 (JP)
(72) Inventor: ITO, Yuki, Saitama-shi, Saitama 330-8508 (JP); NAGATOMO, Yoshiyuki, Saitama-shi, Saitama 330-8508 (JP); TOSHIMORI, Yuto, Naka-shi, Ibaraki 311-0102 (JP)
(74) Representative: Hoffmann Eitle
(86) International application number: PCT/JP2021/012953
(87) International publication number: WO 2021/193940

(57) **Abstract**

An antimicrobial member includes a substrate (111), an underlayer (112) deposited on the substrate (111), and a copper layer (113) formed on a surface of the underlayer (112) which is on a side opposite to the substrate (111) and arranged as an outermost layer, in which the copper layer (113) is formed of copper or a copper alloy, the underlayer (112) is formed of a metal oxide, and the substrate (111) is formed of a flexible resin material. It is preferable that no cracking is confirmed after carrying out a bending test 100 times under a condition where a radius of curvature is 6 mm.

## Description

### TECHNICAL FIELD

The present invention relates to an antimicrobial member with excellent antimicrobial properties and bendability.

In addition, the present invention relates to a low-cost antimicrobial member with excellent antimicrobial properties and durability.

In addition, the present invention relates to a transparent antimicrobial member with excellent transmittance of visible light and excellent antimicrobial properties.

The present application claims priority on Japanese Patent Application No. 2020-057131 filed on March 27, 2020, Japanese Patent Application No. 2020-109482 filed on June 25, 2020, and Japanese Patent Application No. 2020-116807 filed on July 7, 2020, the contents of which are incorporated herein by reference.

### BACKGROUND ART

In general, it is desirable that fixtures such as desks, chairs, and shelves, handrails, doorknobs, and the like used in medical institutions, public facilities, and research facilities with strict hygiene control (for example, for food, cosmetics, pharmaceuticals, and the like) have antimicrobial properties from the viewpoint of preventing infectious diseases and preventing the spreading of viruses and bacteria since there is a concern that the above-described objects may be touched by an unspecified large number of people.

Antimicrobial films (antiviral films) shown in Patent Documents 1 and 2, for example, are proposed as films that impart antimicrobial properties to the surfaces of various products such as fixtures such as desks, chairs, and shelves and handrails and doorknobs.

In Patent Document 1, an antimicrobial film is proposed in which an antimicrobial metal thin film (for example, copper, silver, copper alloy, or silver alloy) is formed on the surface of a flexible polymer film base material.

In Patent Document 2, an antiviral film is proposed in which metal particles formed of Cu and Pd are scattered in island shapes on a film forming a base material.

### PRIOR ART DOCUMENTS

### Patent Document

Patent Document 1: Japanese Unexamined Patent Application, First Publication No. 2010-247450 (A)
Patent Document 2: Japanese Unexamined Patent Application, First Publication No.2018-134753 (A)

### DISCLOSURE OF INVENTION

### Problems to be Solved by the Invention

In the antimicrobial films disclosed in Patent Documents 1 and 2, a resin film was used as the base material and moisture from the resin material forming the base material interfered with the adhesive strength between the base material and the metal thin film in some cases. Therefore, there was a concern that, when the antimicrobial film was bent, the base material and the metal thin film might peel off or cracking might occur in the base material or the metal thin film.

The present invention was made in view of the background of the above-described circumstances and has an object of providing an antimicrobial member with excellent antimicrobial properties and bendability and which is able to be used stably.

In addition, in the antimicrobial films disclosed in Patent Documents 1 and 2, metal is arranged as the outermost surface.

The metal layer formed on the surface of the antimicrobial member is formed of copper, silver, palladium, or the like as described above and the manufacturing cost is comparatively high. In addition, as the thickness of the metal layer becomes thicker, the weight thereof increases, and it is necessary to increase the thickness of the film to ensure rigidity in order to support the metal layer. For this reason, there is a demand to form thinner metal layers.

However, in cases where the metal layer of the outermost layer was thinned to form a film, there were problems in that the metal aggregated such that discoloration occurred, appearance defects were generated, and the durability geatly decreased.

The present invention was made in view of the background of the above-described circumstances and has an object of providing a low-cost antimicrobial member with excellent antimicrobial properties and excellent durability.

In addition, in the antimicrobial films disclosed in Patent Documents 1 and 2, there was a problem in that the surfaces of various products on which this antimicrobial film was arranged, were impossible to see and the design quality was deteriorated because a metal was arranged as the outermost surface thereof and the surfaces had a metallic luster.

In addition, there was a concern that, in a case where the metal layer of the outermost layer was thinned and deposited, depending on the use environment, the metal might aggregate to cause discoloration and appearance defects might be generated.

The present invention was made in view of the background of the above-described circumstances and has an object of providing a transparent antimicrobial member with excellent antimicrobial properties and transmittance of visible light, which suppresses the generation of appearance defects and which is able to be used stably.

### Solutions for Solving the Problems

To solve this problem, an antimicrobial member of an aspect of the present invention (referred to below as the "antimicrobial member of the present invention") includes a substrate, an underlayer deposited on the substrate, and a copper layer formed on a surface of the underlayer which is on a side opposite to the substrate and arranged as an outermost layer, in which the copper layer is formed of copper or a copper alloy, the underlayer is formed of a metal oxide, and the substrate is formed of a flexible resin material or a flexible glass material.

In the antimicrobial member with this configuration, since the copper layer formed of copper or a copper alloy is arranged as the outermost layer, the antimicrobial properties are excellent.

In addition, in a case where the substrate is formed of a flexible resin material, an underlayer formed of a metal oxide is disposed between the substrate and the copper layer; and thereby, moisture from the flexible resin material forming the substrate is suppressed from moving to the copper layer side and adhesive strength between the substrate and the copper layer is ensured. Thus, even in a case where the antimicrobial member of the present invention is bent, it is possible to suppress peeling between the substrate and the copper layer and the generation of cracking and stable use is possible. In addition, in a case where the substrate is formed of a flexible glass material, the underlayer formed of metal oxide is disposed between the substrate and the copper layer; and thereby, adhesive strength between the substrate and the copper layer is ensured. Thus, even in a case where the antimicrobial member of the present invention is bent, it is possible to suppress peeling between the substrate and the copper layer and the generation of cracking and stable use is possible.

The copper layer may be formed of copper or a copper alloy and an oxide film may be formed on the surface thereof.

In addition, in the antimicrobial member of the present invention, a thickness of the copper layer is preferably 5 µm or less.

In this case, since the thickness of the copper layer is suppressed as described above, it is possible to suppress the generation of cracking in the copper layer when the copper layer is bent, and in addition, it is possible to suppress peeling off from the underlayer and it is possible to further improve the bendability.

In addition, in the antimicrobial member of the present invention, the thickness of the copper layer may be 35 nm or less.

In the antimicrobial member with this configuration, since the copper layer formed of copper or a copper alloy is arranged as the outermost layer, the antimicrobial properties are excellent. In addition, since the copper layer has a thickness of 35 nm or less, it is possible to suppress the manufacturing cost to be comparatively low.

Since an underlayer formed of metal oxide is formed between the substrate and the copper layer, even when the thickness of the copper layer is 35 nm or less, it is possible to suppress copper aggregation in the copper layer and it is possible to suppress the generation of appearance defects and the durability is excellent.

The copper layer may be formed of copper or a copper alloy and an oxide film may be formed on the surface thereof.

In addition, in the antimicrobial member of the present invention, the substrate may be a transparent substrate and the metal oxide may be a metal oxide through which visible light is transmitted.

In the transparent antimicrobial member with this configuration, since the copper layer formed of copper or a copper alloy is arranged as the outermost layer, the antimicrobial properties are excellent.

In addition, an underlayer formed of a metal oxide through which visible light is transmitted is formed on the transparent substrate and the thickness of the copper layer formed on a surface of the underlayer on the side opposite to the transparent substrate is 35 nm or less; and thereby, the transmittance of visible light is excellent, it is possible to see the surface of the various products on which the transparent antimicrobial member is arranged, and the design quality is excellent.

The copper layer is formed to have a thickness of 35 nm or less which is thin, but an underlayer formed of metal oxide is formed between the transparent substrate and the copper layer; and thereby, it is possible to suppress copper aggregation in the copper layer and it is possible to suppress the generation of appearance defects and the durability is excellent.

In the antimicrobial member of the present invention, it is preferable that no cracking is confirmed after carrying out a bending test 100 times under a condition where a radius of curvature is 6 mm.

In this case, since cracks do not occur even in a case where the bending test is carried out as described above, it is possible to arrange the antimicrobial member in close contact with the surfaces of products of various shapes and it is possible to impart antimicrobial properties to the surfaces of the products.

Furthermore, in the antimicrobial member of the present invention, the thickness of the underlayer is preferably 500 nm or less.

In this case, since the thickness of the underlayer is suppressed as described above, it is possible to suppress the occurrence of cracking in the underlayer during bending and it is possible to further improve the bendability.

In addition, in the antimicrobial member of the present invention, the thickness of the underlayer is preferably 100 nm or less.

In this case, since the thickness of the underlayer formed of a metal oxide is limited to 100 nm or less, it is possible to further suppress the manufacturing cost to be low.

In addition, in the transparent antimicrobial member of the present invention, the thickness of the underlayer is preferably 50 nm or less.

In this case, it is possible to sufficiently ensure the transmittance of visible light in the underlayer and it is possible to sufficiently see the surface of various products on which this transparent antimicrobial member is arranged.

In addition, in the antimicrobial member of the present invention, the thickness of the substrate is preferably 500 µm or less.

In this case, since the thickness of the substrate is suppressed as described above, the bendability of the substrate itself is ensured and it is possible to further improve the bendability.

Furthermore, in the antimicrobial member of the present invention, the metal oxide that forms the underlayer preferably includes any one or more selected from In oxide, Sn oxide, Zn oxide, Nb oxide, Ti oxide, Al oxide, Ga oxide, W oxide, Mo oxide, Si oxide, Zr oxide, Ta oxide, Y oxide, Ge oxide, Cu oxide, and Ag oxide.

In this case, since the underlayer is formed of the metal oxide described above, moisture from the resin material forming the substrate is reliably suppressed from moving to the copper layer side, it is possible to further improve the adhesive strength between the substrate and the copper layer, and it is possible to further improve the bendability.

In addition, in the antimicrobial member of the present invention, the copper layer is preferably formed of a copper alloy which includes one or more selected from the group consisting of Zn, Sn, Ni, Al, Si, and Mn at a total amount of 0.1 mass% or more and in which an amount of Cu is 45 mass% or more.

In this case, since the amount of Cu in the copper layer is 45 mass% or more, it is possible to sufficiently ensure the antimicrobial properties. In addition, since the copper layer includes one or more selected from the group consisting of Zn, Sn, Ni, Al, Si, and Mn at a total amount of 0.1 mass% or more, it is possible to suppress discoloration in the copper layer.

Furthermore, in the antimicrobial member of the present invention, it is preferable that a Cu oxide film is formed on a surface layer of the copper layer and a molar ratio CuO/Cu₂O of CuO and Cu₂O in the Cu oxide film is CuO/Cu₂O < 1.

In this case, in the Cu oxide film formed on the surface layer of the copper layer, Cu₂O is included at an amount more than that of CuO and it is possible to sufficiently ensure the antimicrobial properties.

In addition, in the antimicrobial member of the present invention, an adhesive layer is preferably provided on a surface of the substrate on a side opposite to the underlayer.

In this case, using the adhesive layer makes it possible to easily arrange the antimicrobial member on the surface of various products.

In addition, in the antimicrobial member of the present invention, the substrate is preferably formed of glass or resin.

In this case, it is possible to produce comparatively large antimicrobial members comparatively easily at a low cost.

In the antimicrobial member of the present invention, a transmittance with respect to light having a wavelength of 550 nm in a laminating direction is preferably 5% or more.

In this case, since the transmittance with respect to light having a wavelength of 550 nm in a laminating direction is 5% or more, it is possible to sufficiently see the surface of various products on which this antimicrobial member is arranged and it is possible to reliably improve the design quality.

### Effects of Invention

According to the present invention, it is possible to provide an antimicrobial member with excellent antimicrobial properties and bendability and which is able to be used stably.

According to the present invention, it is also possible to provide a low-cost antimicrobial member with excellent antimicrobial properties and excellent durability.

In addition, according to the present invention, it is possible to provide a transparent antimicrobial member with excellent antimicrobial properties and transmittance of visible light, which suppresses the generation of appearance defects and which is able to be used stably.

### BRIEF DESCRIPTION OF DRAWINGS

Fig. 1 is an explanatory diagram showing an example of an antimicrobial member in an embodiment of the present invention.
Fig. 2A is an explanatory diagram of a test method for evaluating the bendability in Examples.
Fig. 2B is an explanatory diagram of a test method for evaluating the bendability in Examples.
Fig. 2C is an explanatory diagram of a test method for evaluating the bendability in Examples.
Fig. 3 is an explanatory diagram showing an example of an antimicrobial member in an embodiment of the present invention.
Fig. 4 is an explanatory diagram showing an example of a transparent antimicrobial member in an embodiment of the present invention.

### EMBODIMENTS FOR CARRYING OUT THE INVENTION

### [First Embodiment]

A description will be given of an antimicrobial member which is a first embodiment of the present invention.

The antimicrobial member 110 of the present embodiment is arranged on the surfaces of various products such as fixtures such as desks, chairs, and shelves and handrails and doorknobs and imparts antimicrobial properties to the surfaces of the various products.

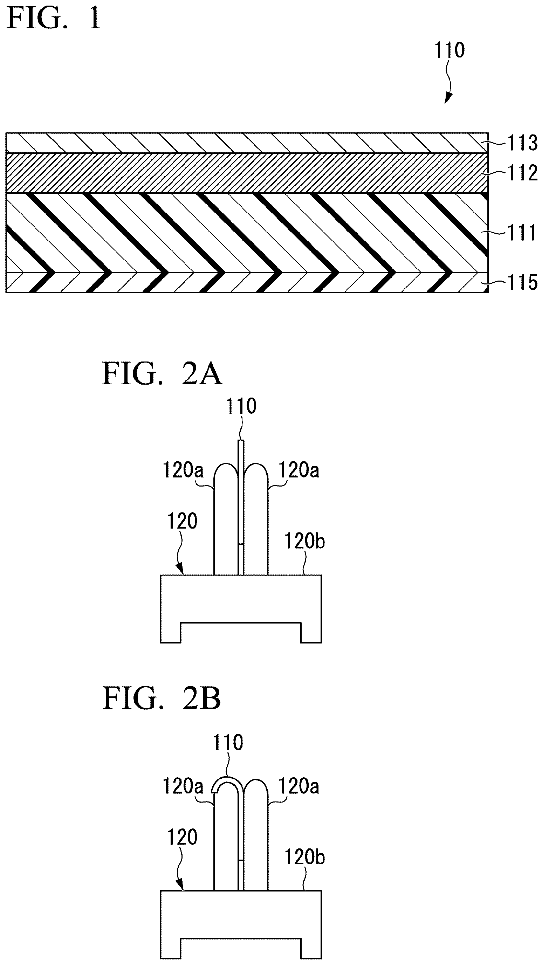

As shown in Fig. 1, the antimicrobial member 110 of the present embodiment includes an underlayer 112 disposed on one surface (top surface in Fig. 1) of a substrate 111, and a copper layer 113 laminated on a surface of the underlayer 112 on the side opposite to the substrate 111.

In the present embodiment, as shown in Fig. 1, an adhesive layer 115 is formed on a surface of the substrate 111 on the side opposite to the underlayer 112.

The antimicrobial member 110 of the present embodiment has excellent bendability and is able to be disposed in close contact with the surfaces of products with various shapes.

Specifically, in the antimicrobial member 110 of the present embodiment, it is preferable that no cracks are confirmed after carrying out a bending test 100 times under a condition where a radius of curvature is 6 mm.

The substrate 111 supports the underlayer 112 and the copper layer 113 and, in the present embodiment, the substrate 111 is formed of a flexible resin material with excellent bendability.

Examples of the flexible resin material forming the substrate 111 include polyethylene phthalate (PET), polyolefin (PO), acrylics, polyvinyl chloride, and the like.

In order to further ensure the bendability of the antimicrobial member 110 in the present embodiment, the thickness of the substrate 111 is preferably 500 µm or less, more preferably 400 µm or less, even more preferably 300 µm or less. On the other hand, in order to ensure the rigidity of the substrate 111, the thickness of the substrate 111 is preferably 10 µm or more, more preferably 25 µm or more, and even more preferably 50 µm or more.

The underlayer 112 is formed of a metal oxide through which visible light is transmitted. The underlayer 112 functions as a barrier layer that suppresses gas components and moisture from the substrate 111 from reaching the copper layer 113 and has an action and effect of suppressing a peeling phenomenon at the interface. In addition, by arranging a metal oxide as the underlayer 112 between the substrate 111 and the copper layer 113, interface energy between the copper layer 113 (Cu) and the underlayer 112 (metal oxide) is lowered, which has the action and effect of suppressing Cu aggregation in the copper layer 113.

As metal oxides forming the underlayer 112, it is preferable to include any one or more selected from In oxide, Sn oxide, Zn oxide, Nb oxide, Ti oxide, Al oxide, Ga oxide, W oxide, Mo oxide, Si oxide, Zr oxide, Ta oxide, Y oxide, Ge oxide, Cu oxide, and Ag oxide.

Specifically, examples thereof include In-Sn oxide, Al-Zn oxide, In-Zn oxide, Zn-Sn oxide, Zn-Sn-Al oxide, Ga-Zn oxide, Zn-Y oxide, Ga-Zn-Y oxide, In-Ga-Zn oxide, and the like.

In order to further ensure the bendability of the antimicrobial member 110 in the present embodiment, the thickness of the underlayer 112 is preferably 500 nm or less, more preferably 400 nm or less, and even more preferably 300 nm or less. On the other hand, in order to further achieve the action and effect as a barrier layer, the thickness of the underlayer 112 is preferably 2 nm or more, more preferably 5 nm or more, and even more preferably 10 nm or more.

The copper layer 113 is formed of copper or a copper alloy. It is known that the copper or copper alloy has an antimicrobial action (including an action to reduce bacteria). By using copper alloys with antimicrobial properties (a bacteria-reducing action) in members that a large number of people come into contact with, it is possible to prevent infections caused by various bacteria and viruses.

In order to further ensure the bendability of the antimicrobial member 110 in the present embodiment, the thickness of the copper layer 113 is preferably 5 µm or less, more preferably 4 µm or less, and even more preferably 3 µm or less. On the other hand, in order to ensure sufficient antimicrobial properties, the thickness of the copper layer 113 is preferably 5 nm or more, more preferably 10 nm or more, and even more preferably 15 nm or more.

In addition, in the present embodiment, the copper layer 113 may be formed of a copper alloy, the copper alloy may include one or more selected from Zn, Sn, Ni, Al, Si, and Mn at a total amount of 0.1 mass% or more and the amount of Cu in the copper alloy may be 45 mass% or more.

By including one or more selected from Zn, Sn, Ni, Al, Si, and Mn at a total amount of 0.1 mass% or more, it is possible to greatly improve the uniformity, durability, and the like of the copper layer 113 when deposited. In order to sufficiently achieve this action and effect, the total amount of one or more selected from Zn, Sn, Ni, Al, Si, and Mn is more preferably set to 0.2 mass% or more and even more preferably set to 0.3 mass% or more.

In addition, when the amount of Cu is 45 mass% or more, it is possible to sufficiently ensure the antimicrobial properties of Cu. The lower limit of the amount of Cu is more preferably 47.5 mass% or more and even more preferably 50 mass% or more.

Furthermore, in the present embodiment, a Cu oxide film may be formed on the outermost surface of the copper layer 113. On the outermost surface of the copper layer 113, the molar ratio CuO/Cu₂O of CuO and Cu₂O is preferably CuO/Cu₂O < 1. That is, it is preferable to include Cu₂O at an amount more than that of CuO. By including Cu₂O at an amount more than that of CuO, it is possible to sufficiently ensure the antimicrobial properties.

The molar ratio CuO/Cu₂O of CuO and Cu₂O is more preferably less than 0.9 and even more preferably less than 0.8.

A description will be given of the method for manufacturing the antimicrobial member 110 according to the present embodiment.

First, the substrate 111 on which the adhesive layer 115 is formed is prepared.

Next, the underlayer 112 is deposited on one surface of the substrate 111 (a surface on the side opposite to the adhesive layer 115) using a sputtering method. At this time, it is preferable to use a sputtering target with a composition corresponding to the oxide that forms the underlayer 112. For example, an oxide sputtering target formed of a metal oxide that forms the underlayer 112 may be used or a reactive sputtering method may be applied by introducing oxygen and using a metal sputtering target for the metal oxide that forms the underlayer 112. In consideration of the electric conductivity and the like of the sputtering target, it is preferable to appropriately select and use DC (direct current) sputtering, RF (radio frequency) sputtering, MF (medium frequency) sputtering, AC (alternating current) sputtering, or the like.

Next, the copper layer 113 is deposited on the deposited underlayer 112 by the sputtering method. At this time, a sputtering target with a composition corresponding to the copper or copper alloy that forms the copper layer 113 is used. At this time, the sputtering conditions are appropriately adjusted such that the thickness of the copper layer 113 becomes a specified thickness. During the sputtering, by measuring the film thickness using a profilometer (DEKTAK-XT) when deposition is carried out for a certain period of time, the sputtering rate is measured and the deposition time is adjusted based on that sputtering rate to carry out the deposition so as to achieve the targeted film thickness.

The steps described above make it possible to manufacture the antimicrobial member 110 of the present embodiment.

According to the antimicrobial member 110 of the present embodiment formed as described above, since the copper layer 113 formed of copper or a copper alloy is arranged as the outermost layer, the antimicrobial properties are excellent.

In addition, since the substrate 111 is formed of a flexible resin material and the underlayer 112 formed of a metal oxide is disposed between the substrate 111 and the copper layer 113, moisture from the flexible resin material forming the substrate 111 is suppressed from moving to the copper layer 113 side and adhesive strength between the substrate 111 and the copper layer 113 is ensured. Thus, even in a case where the antimicrobial member 110 of the present embodiment is bent, it is possible to suppress peeling of the substrate 111 and the copper layer 113 and stable use is possible.

In addition, in a case where the substrate is formed of a flexible glass material, the underlayer formed of metal oxide is disposed between the substrate and the copper layer; and thereby, adhesive strength between the substrate and the copper layer is ensured. Thus, even in a case where the antimicrobial member of the present invention is bent, it is possible to suppress peeling between the substrate and the copper layer and the generation of cracking and stable use is possible.

In addition, in the present embodiment, in a case where no cracking is confirmed after carrying out a bending test 100 times under a condition where a radius of curvature is 6 mm, it is possible to arrange the antimicrobial member in close contact with the surfaces of products of various shapes and it is possible to impart antimicrobial properties to the surfaces of the products.

Furthermore, in the present embodiment, in a case where the thickness of the copper layer 113 is 5 µm or less, in a case where the thickness of the underlayer 112 is 500 nm or less, or in a case where the thickness of the substrate 111 is 500 µm or less, it is possible to further improve the bendability of the antimicrobial member 110.

Furthermore, in the present embodiment, in a case where the copper layer 113 is formed of a copper alloy which includes one or more selected from Zn, Sn, Ni, Al, Si, and Mn at a total amount of 0.1 mass% or more and in which the amount of Cu is 45 mass% or more, it is possible to sufficiently ensure the antimicrobial properties, the durability of the copper layer 113 is improved, and it is possible to suppress the generation of discoloration.

In addition, in the present embodiment, in a case where a Cu oxide film is formed on the surface layer of the copper layer 113 and the molar ratio CuO/Cu₂O of CuO and Cu₂O in this Cu oxide film is CuO/Cu₂O < 1, Cu₂O is included at an amount more than that of CuO and it is possible to sufficiently ensure the antimicrobial properties.

Furthermore, in the present embodiment, in a case where the adhesive layer 115 is provided on the surface of the substrate 111 on the side opposite to the underlayer, it is possible to easily arrange the antimicrobial member 110 on the surfaces of various products by using the adhesive layer 115.

Although embodiments of the present invention were described above, the present invention is not limited thereto and appropriate modification is possible in a range not departing from the technical features of the invention.

For example, in the present embodiment, a description was given of forming an adhesive layer, but an adhesive layer may not be formed.

In addition, the copper or copper alloys forming the copper layer are not limited to the examples described in the embodiments and it is possible to use various copper alloys. For example, the copper alloy may be an alloy obtained by adding Sn, Ni, Al, Pb, Mn, Si, P, and the like to a brass including Cu and Zn as main components. Even in this case, the amount of Cu is preferably set to 45 mass% or more.

### [Second Embodiment]

A description will be given below of an antimicrobial member which is a second embodiment of the present invention.

The antimicrobial member of the present embodiment is, for example, arranged on the surfaces of various products such as fixtures such as desks, chairs, and shelves and handrails and doorknobs and imparts antimicrobial properties to the surfaces of the various products.

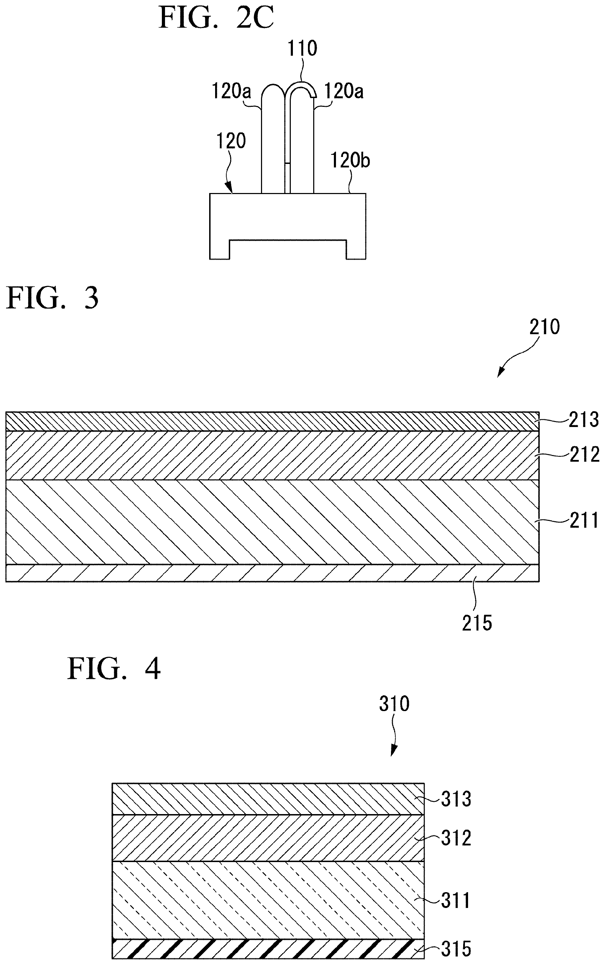

As shown in Fig. 3, an antimicrobial member 210 of the present embodiment includes an underlayer 212 disposed on one surface (upper surface in Fig. 3) of a substrate 211, and a copper layer 213 laminated on a surface of this underlayer 212 on the side opposite to the substrate 211.

In the present embodiment, as shown in Fig. 3, an adhesive layer 215 is formed on the surface of the substrate 211 on the side opposite to the underlayer 212.

The substrate 211 supports the underlayer 212 and the copper layer 213. In the present embodiment, the substrate 211 is preferably formed of a glass material or a resin material.

Examples of the resin material forming the substrate 211 include polyethylene phthalate (PET), polyolefin (PO), acrylics, polyvinyl chloride, and the like. In a case where the substrate 211 formed of these resin materials is used, it is possible to bend the antimicrobial member 210. In particular, by setting the thickness of the substrate 211 formed of the resin material to 300 µm or less, it is possible to further ensure the bendability.

In addition, even in a case where the substrate 211 is formed of a glass material, it is possible to obtain bendability by setting the thickness to 200 µm or less.

The underlayer 212 is formed of a metal oxide. By arranging the metal oxide as the underlayer 212 between the substrate 211 and the copper layer 213, interface energy between the copper layer 213 (Cu) and the underlayer 212 (metal oxide) is lowered; and thereby, the action and effect of suppressing Cu aggregation in the copper layer 213 are obtained. In addition, the underlayer 212 functions as a barrier layer that suppresses gas components and moisture from the substrate 211 from reaching the copper layer 213 and has an action and effect of suppressing a peeling phenomenon at the interface.

As metal oxides forming the underlayer 212, it is preferable to include any one or more selected from In oxide, Sn oxide, Zn oxide, Nb oxide, Ti oxide, Al oxide, Ga oxide, W oxide, Mo oxide, Si oxide, Zr oxide, Ta oxide, Y oxide, Ge oxide, Cu oxide, and Ag oxide.

Specifically, examples thereof include In-Sn oxide, Al-Zn oxide, In-Zn oxide, Zn-Sn oxide, Zn-Sn-Al oxide, Ga-Zn oxide, Zn-Y oxide, Ga-Zn-Y oxide, In-Ga-Zn oxide, and the like.

In addition, in a case of attempting to reduce the manufacturing cost, the upper limit of the thickness of the underlayer 212 is preferably set to 100 nm or less and more preferably set to 50 nm or less.

On the other hand, in order to sufficiently achieve the action and effect of the underlayer 212, the lower limit of the thickness of the underlayer 212 is preferably set to 5 nm or more, more preferably set to 8 nm or more, and even more preferably set to 10 nm or more.

The copper layer 213 is formed of copper or a copper alloy. It is known that the copper or copper alloy has an antimicrobial action (including an action to reduce bacteria). By using copper alloys with antimicrobial properties (a bacteria-reducing action) in members that an unspecified large number of people come into contact with, it is possible to prevent infections caused by various bacteria and viruses.

When the thickness of the copper layer 213 becomes thicker than 30 nm, the manufacturing cost will increase and the weight will also increase without further improving the antimicrobial properties. For this reason, in the present embodiment, the upper limit of the thickness of the copper layer 213 is set to 30 nm or less. The upper limit of the thickness of the copper layer 213 is preferably set to 27 nm or less and more preferably set to 25 nm or less.

On the other hand, when the thickness of the copper layer 213 is excessively thin, there is a concern that the ratio of the surface to the volume of the copper layer 213 may become comparatively high and energetically unstable, the copper may easily aggregate, and the durability may be reduced. For this reason, the lower limit of the thickness of the copper layer 213 is preferably set to 2 nm or more, more preferably set to 5 nm or more, and even more preferably set to 10 nm or more.

In addition, in the present embodiment, the copper layer 213 may be formed of a copper alloy, the copper alloy may include one or more selected from Zn, Sn, Ni, Al, Si, and Mn at a total amount of 0.1 mass% or more and the amount of Cu in the copper alloy may be 45 mass% or more.

By including one or more selected from Zn, Sn, Ni, Al, Si, and Mn at a total amount of 0.1 mass% or more, it is possible to greatly improve the uniformity, durability, and the like of the copper layer 213 when deposited. In order to sufficiently achieve this action and effect, the total amount of one or more selected from Zn, Sn, Ni, Al, Si, and Mn is more preferably set to 0.2 mass% or more and even more preferably set to 0.3 mass% or more.

In addition, when the amount of Cu is 45 mass% or more, it is possible to sufficiently ensure the antimicrobial properties of Cu. The lower limit of the amount of Cu is more preferably 47.5 mass% or more and even more preferably 50 mass% or more.

Furthermore, in the present embodiment, a Cu oxide film may be formed on the outermost surface of the copper layer 213. On this outermost surface of the copper layer 213, the molar ratio CuO/Cu₂O of CuO and Cu₂O is preferably CuO/Cu₂O < 1. That is, preferably, Cu₂O is included at an amount more than that of CuO. By including Cu₂O at an amount more than that of CuO, it is possible to sufficiently ensure the antimicrobial properties.

The molar ratio CuO/Cu₂O of CuO and Cu₂O is more preferably less than 0.9 and even more preferably less than 0.8.

A description will be given below of the method for manufacturing the antimicrobial member 210 according to the present embodiment.

First, the substrate 211 on which the adhesive layer 215 is formed is prepared.

Next, the underlayer 212 is deposited on one surface of the substrate 211 (the surface on the side opposite to the adhesive layer 215) by a sputtering method. At this time, it is preferable to use a sputtering target with a composition corresponding to the oxide that forms the underlayer 212. For example, an oxide sputtering target formed of a metal oxide that forms the underlayer 212 may be used or a reactive sputtering method may be applied by introducing oxygen and using a metal sputtering target for the metal oxide that forms the underlayer 212. In consideration of the electric conductivity and the like of the sputtering target, it is preferable to appropriately select and use DC (direct current) sputtering, RF (radio frequency) sputtering, MF (medium frequency) sputtering, AC (alternating current) sputtering, or the like.

Next, the copper layer 213 is deposited on the deposited underlayer 212 by the sputtering method. At this time, a sputtering target with a composition corresponding to the Cu or Cu alloy that forms the copper layer 213 is used. At this time, the sputtering conditions are appropriately adjusted such that the thickness of the copper layer 213 becomes a specified thickness. During the sputtering, by measuring the film thickness using a profilometer (DEKTAK-XT) when deposition is carried out for a certain period of time, the sputtering rate is measured and the deposition time is adjusted based on that sputtering rate to carry out the deposition so as to achieve the targeted film thickness.

The steps described above make it possible to manufacture the antimicrobial member 210 of the present embodiment.

According to the antimicrobial member 210 of the present embodiment formed as described above, since the copper layer 213 formed of copper or a copper alloy is arranged as the outermost layer, the antimicrobial properties are excellent.

In addition, since the thickness of the copper layer 213 is set to 30 nm or less, it is possible to suppress the manufacturing cost to be comparatively low.

Although the thickness of the copper layer 213 is formed to be as thin as 30 nm or less, the underlayer 212 formed of metal oxide is formed between the substrate 211 and the copper layer 213; and thereby, it is possible to suppress copper aggregation in the copper layer 213, it is possible to suppress the generation of appearance defects, and the durability is excellent.

In the present embodiment, in a case where the thickness of the underlayer 212 is 100 nm or less, it is possible to further suppress the manufacturing cost to be low.

Furthermore, in the present embodiment, in a case where the metal oxide that forms the underlayer 212 includes any one or more selected from In oxide, Sn oxide, Zn oxide, Nb oxide, Ti oxide, Al oxide, Ga oxide, W oxide, Mo oxide, Si oxide, Zr oxide, Ta oxide, Y oxide, Ge oxide, Cu oxide, and Ag oxide, it is possible to further suppress the aggregation of copper in the copper layer 213, it is possible to further suppress the generation of appearance defects, and it is possible to further improve the durability.

In addition, in the present embodiment, in a case where the copper layer 213 is formed of a copper alloy which includes one or more selected from Zn, Sn, Ni, Al, Si, and Mn at a total amount of 0.1 mass% or more and in which the amount of Cu is 45 mass% or more, it is possible to sufficiently ensure the antimicrobial property, the durability of the copper layer 213 is further improved, and it is possible to suppress the generation of discoloration.

Furthermore, in a case where the substrate 211 is formed of glass or resin in the present embodiment, it is possible to produce the comparatively large antimicrobial member 210 comparatively easily at a low cost.

In addition, in the present embodiment, in a case where a Cu oxide film is formed on the surface layer of the copper layer 213 and the molar ratio CuO/Cu₂O of CuO and Cu₂O in this Cu oxide film is CuO/Cu₂O < 1, Cu₂O is included at an amount more than that of CuO and it is possible to sufficiently ensure the antimicrobial property.

Furthermore, in the present embodiment, in a case where the adhesive layer 215 is provided on the surface of the substrate 211 on the side opposite to the underlayer, it is possible to easily arrange the antimicrobial member 210 on the surfaces of various products using the adhesive layer 215.

Although embodiments of the present invention were described above, the present invention is not limited thereto and appropriate modification is possible in a range not departing from the technical features of the invention.

For example, in the present embodiment, a description was given of forming an adhesive layer, but an adhesive layer may not be formed.

In addition, the copper or copper alloys forming the copper layer are not limited to the examples described in the embodiments and it is possible to use various copper alloys. For example, the copper alloy may be an alloy obtained by adding Sn, Ni, Al, Pb, Mn, Si, P, and the like to a brass including Cu and Zn as main components. Even in this case, the amount of Cu is preferably set to 45 mass% or more.

### [Third Embodiment]

A description will be given below of a transparent antimicrobial member which is a third embodiment of the present invention.

The transparent antimicrobial member of the present embodiment is arranged on the surfaces of various products such as fixtures such as desks, chairs, and shelves and handrails and doorknobs and imparts antimicrobial properties to the surfaces of the various products.

As shown in Fig. 4, a transparent antimicrobial member 310 of the present embodiment includes an underlayer 312 disposed on one surface (the upper surface in Fig. 4) of a transparent substrate 311, and a copper layer 313 laminated on a surface of this underlayer 312 on the side opposite to the transparent substrate 311.

In the present embodiment, as shown in Fig. 4, an adhesive layer 315 is formed on the surface of the transparent substrate 311 on the side opposite to the underlayer 312.

The transparent substrate 311 is preferably formed of a material having high transmittance of visible light, such as a glass material or a resin material.

Examples of the resin material forming the transparent substrate 311 include polyethylene phthalate (PET), polyolefin (PO), acrylics, polyvinyl chloride, and the like. In a case where the transparent substrate 311 formed of these resin materials is used, it is possible to bend the transparent substrate 311. In particular, by setting the thickness of the transparent substrate 311 formed of a resin material to 300 µm or less, it is possible to further ensure the bendability.

In addition, even in a case where the transparent substrate 311 is formed of glass material, it is possible to obtain bendability by setting the thickness to 200 µm or less.

Although there is no particular limit to the lower limit of the thickness of the transparent substrate 311, from the viewpoint of ensuring rigidity, the thickness is preferably 10 µm or more and more preferably 20 µm or more.

The underlayer 312 is formed of a metal oxide through which visible light is transmitted. By arranging the metal oxide as the underlayer 312 between the transparent substrate 311 and the copper layer 313, interface energy between the copper layer 313 (Cu) and the underlayer 312 (metal oxide) is lowered; and thereby, the action and effect of suppressing Cu aggregation in the copper layer 313 are obtained. In addition, the underlayer 312 functions as a barrier layer that suppresses gas components and moisture from the transparent substrate 311 from reaching the copper layer 313 and has an action and effect of suppressing a peeling phenomenon at the interface.

As metal oxides forming the underlayer 312, it is preferable to include one or more selected from In oxide, Sn oxide, Zn oxide, Nb oxide, Ti oxide, Al oxide, Ga oxide, W oxide, Mo oxide, Si oxide, Zr oxide, Ta oxide, Y oxide, Ge oxide, Cu oxide, and Ag oxide.

Specifically, examples thereof include In-Sn oxide, Al-Zn oxide, In-Zn oxide, Zn-Sn oxide, Zn-Sn-Al oxide, Ga-Zn oxide, Zn-Y oxide, Ga-Zn-Y oxide, In-Ga-Zn oxide, and the like.

These metal oxides have excellent visible light transmittance.

In addition, in order to sufficiently ensure transmittance of visible light in the underlayer 312, the upper limit of the thickness of the underlayer 312 is preferably set to 100 nm or less and more preferably set to 50 nm or less.

On the other hand, in order to sufficiently achieve the action and effect as the underlayer 312, the lower limit of the thickness of the underlayer 312 is preferably set to 3 nm or more, more preferably set to 4 nm or more, and even more preferably set to 5 nm or more.

The copper layer 313 is formed of copper or a copper alloy. It is known that the copper or copper alloy has an antimicrobial action (including an action to reduce bacteria). By using copper alloys with antimicrobial properties (a bacteria-reducing action) in members that an unspecified large number of people come into contact with, it is possible to prevent infections caused by various bacteria and viruses.

In the present embodiment, the upper limit of the thickness of the copper layer 313 is set to 35 nm or less. Due to this, it is possible to ensure the transmittance of visible light in the copper layer 313. In order to further ensure the transmittance of visible light in the copper layer 313, the upper limit of the thickness of the copper layer 313 is preferably set to 30 nm or less and more preferably set to 25 nm or less.

On the other hand, when the thickness of the copper layer 313 is excessively thin, there is a concern that the ratio of the surface to the volume of the copper layer 313 may become comparatively high and energetically unstable, the copper may easily aggregate, and the durability may be reduced. For this reason, the lower limit of the thickness of the copper layer 313 is preferably set to 2 nm or more, more preferably set to 3 nm or more, and even more preferably set to 4 nm or more.

In addition, in the present embodiment, the copper layer 313 may be formed of a copper alloy, the copper alloy may include one or more selected from Zn, Sn, Ni, Al, Si, and Mn at a total amount of 0.1 mass% or more and the amount of Cu in the copper alloy may be 45 mass% or more.

By including one or more selected from Zn, Sn, Ni, Al, Si, and Mn at a total amount of 0.1 mass% or more, it is possible to greatly improve the uniformity, durability, and the like of the copper layer 313 when deposited. In order to sufficiently achieve this action and effect, the total amount of one or more selected from Zn, Sn, Ni, Al, Si, and Mn is more preferably set to 0.2 mass% or more and even more preferably set to 0.3 mass% or more.

In addition, when the amount of Cu is 45 mass% or more, it is possible to sufficiently ensure the antimicrobial properties of Cu. The lower limit of the amount of Cu is more preferably 47.5 mass% or more and even more preferably 50 mass% or more.

Furthermore, in the present embodiment, a Cu oxide film may be formed on the outermost surface of the copper layer 313. In the outermost surface of this copper layer 313, the molar ratio CuO/Cu₂O of CuO and Cu₂O is preferably CuO/Cu₂O < 1. That is, it is preferable to include Cu₂O at an amount more than that of CuO. By including Cu₂O at an amount more than that of CuO, it is possible to sufficiently ensure the antimicrobial properties.

The molar ratio CuO/Cu₂O of CuO and Cu₂O is preferably less than 0.9 and more preferably less than 0.8.

In the transparent antimicrobial member 310 of the present embodiment, the transmittance with respect to light having the wavelength of 550 nm in the laminating direction is preferably 5% or more.

In the transparent antimicrobial member 310 of the present embodiment, the transmittance with respect to light having the wavelength of 550 nm in the laminating direction is preferably 7% or more and more preferably 10% or more.

A description will be given below of the method for manufacturing the transparent antimicrobial member 310 according to the present embodiment.

First, the transparent substrate 311 on which an adhesive layer 315 is formed is prepared.

Next, the underlayer 312 is deposited on one surface of the transparent substrate 311 (the surface on the side opposite to the adhesive layer 315) by a sputtering method. At this time, it is preferable to use a sputtering target with a composition corresponding to the oxide that forms the underlayer 312. For example, an oxide sputtering target formed of a metal oxide that forms the underlayer 312 may be used or a reactive sputtering method may be applied by introducing oxygen and using a metal sputtering target for the metal oxide that forms the underlayer 312. In consideration of the electric conductivity and the like of the sputtering target, it is preferable to appropriately select and use DC (direct current) sputtering, RF (radio frequency) sputtering, MF (medium frequency) sputtering, AC (alternating current) sputtering, or the like.

Next, a copper layer 313 is deposited on the deposited underlayer 312 by a sputtering method. At this time, a sputtering target with a composition corresponding to the Cu or Cu alloy that forms the copper layer 313 is used. At this time, the sputtering conditions are appropriately adjusted such that the thickness of the copper layer 313 becomes a specified thickness. During the sputtering, by measuring the film thickness using a profilometer (DEKTAK-XT) when deposition is carried out for a certain period of time, the sputtering rate is measured and the deposition time is adjusted based on that sputtering rate to carry out the deposition so as to achieve the targeted film thickness.

The steps described above make it possible to manufacture the transparent antimicrobial member 310 of the present embodiment.

According to the transparent antimicrobial member 310 of the present embodiment formed as described above, since the copper layer 313 formed of copper or a copper alloy is arranged as the outermost layer, the antimicrobial properties are excellent.

In addition, since the underlayer 312 formed of a metal oxide through which visible light is transmitted is formed on the transparent substrate 311 and the thickness of the copper layer 313 is set to 35 nm or less, the transmittance of visible light is excellent, it is possible to see the surfaces of various products on which the transparent antimicrobial member 310 is arranged, and the design quality is excellent.

Although the thickness of the copper layer 313 is formed to be 35 nm or less which is thin, the underlayer 312 formed of metal oxide is formed between the transparent substrate 311 and the copper layer 313; and thereby, it is possible to suppress copper aggregation in the copper layer 313 and it is possible to suppress the generation of appearance defects.

In the present embodiment, in a case where the transmittance with respect to light having the wavelength of 550 nm in the laminating direction is 5% or more, it is possible to sufficiently see the surfaces of various products on which this transparent antimicrobial member 310 is arranged and it is possible to reliably improve the design quality.

Furthermore, in the present embodiment, in a case where the metal oxide that forms the underlayer 312 includes one or more selected from In oxide, Sn oxide, Zn oxide, Nb oxide, Ti oxide, Al oxide, Ga oxide, W oxide, Mo oxide, Si oxide, Zr oxide, Ta oxide, Y oxide, Ge oxide, Cu oxide, and Ag oxide, the transmittance of visible light in the underlayer 312 is sufficiently ensured and it is possible to sufficiently see the surfaces of various products on which this transparent antimicrobial member 310 is arranged.

In addition, in the present embodiment, in a case where the thickness of the underlayer 312 is 50 nm or less, it is possible to sufficiently ensure the transmittance of visible light in the underlayer 312 and it is possible to sufficiently see the surfaces of various products on which this transparent antimicrobial member 310 is arranged.

Furthermore, in the present embodiment, in a case where the copper layer 313 is formed of a copper alloy which includes one or more selected from Zn, Sn, Ni, Al, Si, and Mn at a total amount of 0.1 mass% or more and in which the amount of Cu is 45 mass% or more, it is possible to sufficiently ensure the antimicrobial property, the durability of the copper layer 313 is further improved, and it is possible to suppress the generation of discoloration.

In addition, in the present embodiment, in a case where a Cu oxide film is formed on the surface layer of the copper layer 313 and the molar ratio CuO/Cu₂O of CuO and Cu₂O in this Cu oxide film is CuO/Cu₂O < 1, Cu₂O is included at an amount more than that of CuO and it is possible to sufficiently ensure the antimicrobial properties.

Furthermore, in the present embodiment, in a case where the adhesive layer 315 is provided on the surface of the transparent substrate 311 on the side opposite to the underlayer, it is possible to easily arrange the transparent antimicrobial member 310 on the surfaces of various products using the adhesive layer 315.

Although embodiments of the present invention were described above, the present invention is not limited thereto and appropriate modification is possible in a range not departing from the technical features of the invention.

For example, in the present embodiment, a description was given of forming an adhesive layer, but an adhesive layer may not be formed.

In addition, the copper or copper alloys forming the copper layer are not limited to the examples described in the embodiments and it is possible to use various copper alloys. For example, the copper alloy may be an alloy obtained by adding Sn, Ni, Al, Pb, Mn, Si, P, and the like to a brass including Cu and Zn as main components. Even in this case, the amount of Cu is preferably set to 45 mass% or more.

### EXAMPLES

### [First Example]

A description will be given below of the results of confirmation experiments performed to confirm the effects of the present invention.

The sputtering target used when depositing the copper layer was manufactured according to the following procedure.

First, as raw materials, a Cu raw material having a purity of 99.9 mass% or higher and metal raw materials having a purity of 99 mass% or higher were weighed to have a predetermined composition. Next, in a melting furnace, the Cu raw material was melted in a high vacuum or in an inert gas atmosphere, and predetermined amounts of the metal raw materials were added to the obtained molten copper. Thereafter, the resultant metal was melted in a vacuum or an inert gas atmosphere to produce a melt-cast ingot. The obtained ingot was subjected to cold rolling, and then a heat treatment was carried out in air, for example, by holding at 600°C for 2 hours and then machining was carried out to produce a disc shape having a diameter of 152.4 mm and a thickness of 6 mm. Thereby, a sputtering target for forming a copper layer was manufactured.

For the sputtering targets used when depositing the underlayer, the sputtering targets listed in Tables 1 and 2, purchased from Kojundo Chemical Laboratory Co., Ltd., were used.

Each of these sputtering targets was joined to a backing plate made of oxygenfree copper by soldering, the resultant target was mounted in a DC magnetron sputtering apparatus, and the inside of the DC magnetron sputtering apparatus was exhausted by a vacuum exhaust system to obtain a degree of vacuum of 5 × 10⁻⁵ Pa or less, then, Ar gas was introduced, and plasma was generated between the cleaned substrates shown in Tables 1 and 2, which were arranged in parallel to the target, and the target described above. Thereby, the underlayer and the copper layer were formed. The deposition conditions for the underlayer and the deposition conditions for the copper layer are as shown below.

### <Deposition Conditions of Underlayer>

Gas used: Ar + 2 volume% of oxygen
Gas pressure: 0.67 Pa
Sputtering power: DC 300 W
Distance between target and substrate: 70 mm

### <Deposition Conditions of Copper Layer>

Peak degree of vacuum: 5×10⁻⁵ Pa or less
Gas used: Ar
Gas pressure: 0.67 Pa
Sputtering power: DC 200W
Distance between target and substrate: 70 mm

The antimicrobial members obtained as described above were evaluated for the items below.

### (Component Composition of Sputtering Target for Forming Copper Layer)

Samples for analysis were taken from the sputtering target for forming the copper layer and the components were measured by ICP emission spectroscopy. It was confirmed that the composition of the sputtering target for forming the copper layer was the same as the composition of the deposited copper layer.

As the compositions of "Others" in the table, it is possible to use Pb, Mg, Zr, Te, Cr, Fe, Co, P, and the like.

### (Measurement of Film Thickness)

The thicknesses of the underlayer and the copper layer were confirmed by observing the cross-sections of the underlayer and the copper layer with a transmission electron microscope (TEM), and it was confirmed that the layers were deposited to have the targeted thicknesses. For preparing samples for TEM observation, for example, it is possible to use a cross-section polisher (CP) or a focused ion beam (FIB).

### (Bendability)

The antimicrobial member was held in an environment at a temperature of 30°C and a relative humidity of 90% for 24 hours, then a bending test was carried out. In the bending test, the antimicrobial member was bent to be alternately concave and convex 100 times under a condition where a radius of curvature was 6 mm. The antimicrobial member was observed after the bending test and the presence or absence of peeling and internal peeling (cracking) was confirmed.

Specifically, as shown in Fig. 2A, the antimicrobial member 110 was interposed between a pair of holding elements 120a erected on a base portion 120b of a bending test jig 120 and, as shown in Fig. 2B and Fig. 2C, bending was performed once to the left and once to the right, for 100 times each. The tips of the pair of holding elements 120a were each a circular arc shape having a radius of curvature of 6 mm in the cross-section. In other words, the bending test was performed in a state where the antimicrobial member 110 was set so as to be positioned at the tip of the holding elements 120a having a radius of curvature of 6 mm.

For the presence or absence of cracking, the test was performed five times, and a case where cracks observed from the substrate side were generated all five times was evaluated as "C", a case where cracks were generated one to four times was evaluated as "B", and a case where cracks were not confirmed was evaluated as "A".

### (Evaluation of Antimicrobial Activity)

The antimicrobial activity of the copper and copper alloys of the sputtering targets which were to be deposited on the surface was evaluated as the antimicrobial activity.

The surface of the target used for sputtering was polished with a sandpaper #1000 and a new surface was exposed, then, the surface oxidation state was adjusted to make a sample. A stainless steel plate which did not include a copper component was used as the reference material. Thereafter, the surface of each specimen was inoculated with a predetermined amount of Staphylococcus aureus in accordance with JIS Z 2801 and the viable bacteria count (cfu) was quantified after 30 minutes. The number of samples was 3, and using the quantified viable bacteria count, the reduction rate of the obtained bacteria was calculated from the following equation. A case where the bacteria reduction rate was more than 2 was evaluated as "A", a case where the bacteria reduction rate was more than 0 and 2 or less was evaluated as "B", and a case where the bacteria reduction rate was 0 or less was evaluated as "C".

Bacteria reduction rate = -log (viable bacteria count (cfu) of specimen after 2 hours/initial inoculation count (cfu))

### (Form of Oxide Present on Surface)

Samples equivalent to sputtering targets were prepared and were subjected to a heat treatment in an oxidizing and reducing atmosphere. Thereby, the form of the oxides present on the surface was changed as shown in Table 3. Due to this, the ratio of CuO and Cu₂O in the surface oxide was changed and antimicrobial activity thereof was measured. The surface oxide state was measured by XPS analysis.

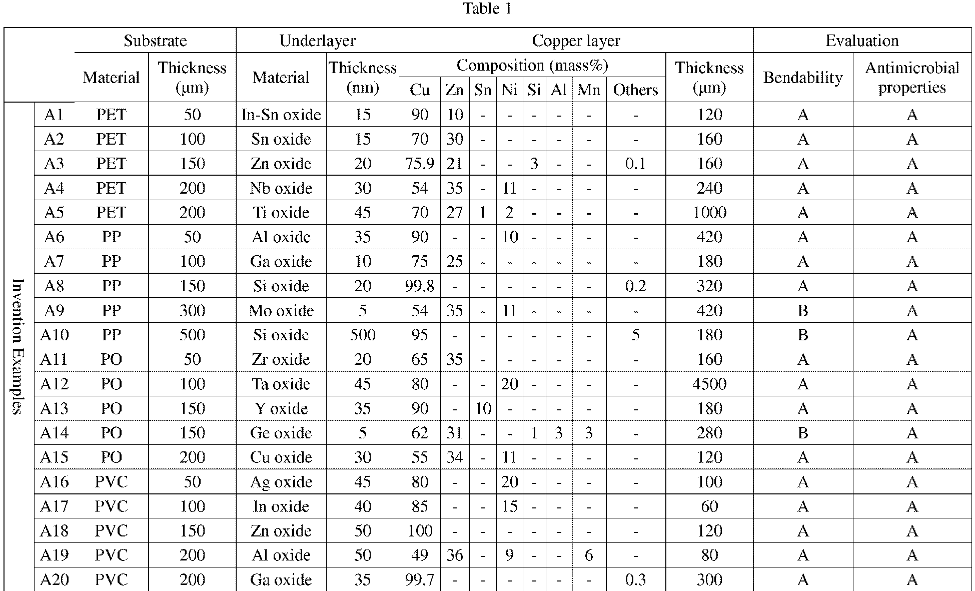

**Table 2**

| | | Substrate | | Underlayer | | Copper layer | | | | | | | | | Evaluation | |
|---|---|---|---|---|---|---|---|---|---|---|---|---|---|---|---|---|
| | | Material | Thickness (µm) | Material | Thickness (nm) | Composition (mass%) | | | | | | | | Thickness (nm) | Bendability | Antimicrobial properties |
| | | | | | | Cu | Zn | Sn | Ni | Si | Al | Mn | Others | | | |
| Invention Examples | A21 | Acrylic | 50 | Cu oxide | 40 | 85 | 15 | - | - | - | - | - | - | 360 | A | A |
| | A22 | Acrylic | 100 | Nb oxide | 35 | 99 | - | - | - | - | 1 | - | - | 160 | A | A |
| | A23 | Acrylic | 150 | Ti oxide | 15 | 95 | - | 5 | - | - | - | - | - | 2000 | A | A |
| | A24 | Acrylic | 150 | In oxide | 50 | 85 | - | - | 15 | - | - | - | - | 40 | A | A |
| | A25 | Acrylic | 300 | Cu oxide | 10 | 90 | 5 | 5 | - | - | - | - | - | 140 | B | A |
| | A26 | Polyimide | 50 | Sn oxide | 200 | 60 | - | - | 40 | - | - | - | - | 360 | A | A |
| | A27 | Polyimide | 150 | In oxide | 35 | 95 | 5 | - | - | - | - | - | - | 180 | A | A |
| | A28 | Polyimide | 150 | Zn oxide | 40 | 100 | - | - | - | - | - | - | - | 220 | A | A |
| | A29 | Polyimide | 150 | W oxide | 10 | 54 | 34 | - | 12 | - | - | - | - | 80 | A | A |
| | A30 | Polyimide | 200 | Si oxide | 20 | 90 | - | - | 10 | - | - | - | - | 180 | A | A |
| Comparative Example A1 | | PET | 200 | - | - | 100 | | | | | | | | 1000 | C | A |

**Table 3**

| | | Substrate | | Underlayer | | Copper layer | | | | | | | | | Surface XPS analysis | |
|---|---|---|---|---|---|---|---|---|---|---|---|---|---|---|---|---|
| | | Material | Thickness (µm) | Material | Thickness (nm) | Composition (mass%) | | | | | | | | Thickness (nm) | CuO/Cu₂O | Antimicrobial properties |
| | | | | | | Cu | Zn | Sn | Ni | Si | Al | Mn | Others | | | |
| Invention Examples | A28 | Polyimide | 150 | Zn oxide | 40 | 100 | - | - | - | - | - | - | - | 220 | <1 | A |
| | A31 | Polyimide | 150 | Zn oxide | 40 | 100 | - | - | - | - | - | - | - | 220 | ≥1 | B |

In Comparative Example A1, a copper layer having a thickness of 1000 nm was deposited directly on a substrate (PET resin substrate) without forming an underlayer, and the bendability was "C". It is assumed that this was because moisture from the substrate moved to the copper layer side and the adhesive strength between the substrate and the copper layer decreased.

In contrast, in Invention Examples A1 to A30, an underlayer formed of metal oxide was formed between the substrate formed of flexible resin material, the copper layer, and the bendability was "A" to "B". In addition, in the copper layers having the compositions shown in Tables 1 and 2, the bacteria reduction rate was more than 2 in all cases and the evaluation was "A".

Furthermore, as shown in Table 3, in Invention Example A28, the molar ratio CuO/Cu₂O of CuO and Cu₂O in the Cu oxide film formed on the surface layer of the copper layer was less than 1, and it was confirmed that the antimicrobial property of Invention Example A28 was improved in comparison with Invention Example A31 in which the molar ratio CuO/Cu₂O of CuO and Cu₂O in the Cu oxide film was 1 or more.

From the above, it was confirmed that, according to the Invention Examples, it is possible to provide an antimicrobial member with excellent antimicrobial properties and bendability and which is able to be used stably.

### [Second Example]

A description will be given below of the results of confirmation experiments performed to confirm the effects of the present invention.

The sputtering target used when depositing the copper layer was manufactured according to the following procedure.

First, as raw materials, a Cu raw material having a purity of 99.9 mass% or higher and metal raw materials having a purity of 99 mass% or higher were weighed to have a predetermined composition. Next, in a melting furnace, the Cu raw material was melted in a high vacuum or in an inert gas atmosphere, and predetermined amounts of the metal raw materials were added to the obtained molten copper. Thereafter, the resultant metal was melted in a vacuum or an inert gas atmosphere to produce a melt-cast ingot. The obtained ingot was subjected to cold rolling, and then a heat treatment was carried out in air, for example, by holding at 600°C for 2 hours and then machining was carried out to produce a disc shape having a diameter of 152.4 mm and a thickness of 6 mm. Thereby, a sputtering target for forming a copper layer was manufactured.

For the sputtering targets used when depositing the underlayer, the sputtering targets listed in Tables 4 and 5, purchased from Kojundo Chemical Laboratory Co., Ltd., were used.

Each of these sputtering targets was joined to a backing plate made of oxygenfree copper by soldering, the resultant target was mounted in a DC magnetron sputtering apparatus, and the inside of the DC magnetron sputtering apparatus was exhausted by a vacuum exhaust system to obtain a degree of vacuum of 5 × 10⁻⁵ Pa or less, then, Ar gas was introduced, and plasma was generated between the cleaned substrates shown in Tables 4 and 5, which were arranged in parallel to the target, and the target described above. Thereby, the underlayer and the copper layer were formed. The deposition conditions for the underlayer and the deposition conditions for the copper layer are as shown below.

### <Deposition Conditions of Underlayer>

Gas used: Ar + 2 volume% of oxygen
Gas pressure: 0.67 Pa
Sputtering power: DC 300 W
Distance between target and substrate: 70 mm

### <Deposition Conditions of Copper Layer>

Peak degree of vacuum: 5×10⁻⁵ Pa or less
Gas used: Ar
Gas pressure: 0.67 Pa
Sputtering power: DC 200W
Distance between target and substrate: 70 mm

The antimicrobial members obtained as described above were evaluated for the items below.

### (Component Composition of Sputtering Target for Forming Copper Layer)

Samples for analysis were taken from the sputtering target for forming the copper layer and the components were measured by ICP emission spectroscopy. It was confirmed that the composition of the sputtering target for forming the copper layer was the same as the composition of the deposited copper layer.

As the compositions of "Others" in the table, it is possible to use Pb, Mg, Zr, Te, Cr, Fe, Co, P, and the like.

### (Measurement of Film Thickness)

The thicknesses of the underlayer and the copper layer were confirmed by observing the cross-sections of the underlayer and the copper layer with a transmission electron microscope (TEM) and it was confirmed that the layers were deposited to have the targeted thicknesses. For preparing samples for TEM observation, for example, it is possible to use a cross-section polisher (CP) or a focused ion beam (FIB).

### (Durability Test)

The antimicrobial member was held in an environment at a temperature of 60°C and 90% humidity for 250 hours as a constant temperature and humidity test and the change in color of the surface appearance after the test was confirmed. The change in color was visually determined. A case where the discolored area covered the entire surface of a sample having a size of 40 mm × 40 mm was evaluated as "D", a case where the discolored area was half or less of the entire surface was evaluated as "B", and a case where the discolored area was not seen was evaluated as "A".

### (Evaluation of Antimicrobial Activity)

The antimicrobial activity of the copper and copper alloys of the sputtering targets which were to be deposited on the surface was evaluated as the antimicrobial activity.

The surface of the target used for sputtering was polished with a sandpaper #1000 and a new surface was exposed, then, the surface oxidation state was adjusted to make a sample. Thereafter, the surface of each specimen was inoculated with a predetermined amount of Staphylococcus aureus in accordance with JIS Z 2801 and the viable bacteria count (cfu) was quantified after 2 hours. The number of samples was 3, and using the quantified viable bacteria count, the reduction rate of the obtained bacteria was calculated from the following equation. A case where the bacteria reduction rate was more than 2 was evaluated as "A", a case where the bacteria reduction rate was more than 0 and 2 or less was evaluated as "B", and a case where the bacteria reduction rate was 0 or less was evaluated as "C".

Bacteria reduction rate = -log (viable bacteria count (cfu) of specimen after 2 hours/initial inoculation count (cfu))

### (Form of Oxide Present on Surface)

Samples equivalent to sputtering targets were prepared and were subjected to a heat treatment in an oxidizing and reducing atmosphere. Thereby, the form of the oxides present on the surface was changed as shown in Table 6. Due to this, the ratio of CuO and Cu₂O in the surface oxide was changed and antimicrobial activity thereof was measured. The surface oxide state was measured by XPS analysis.

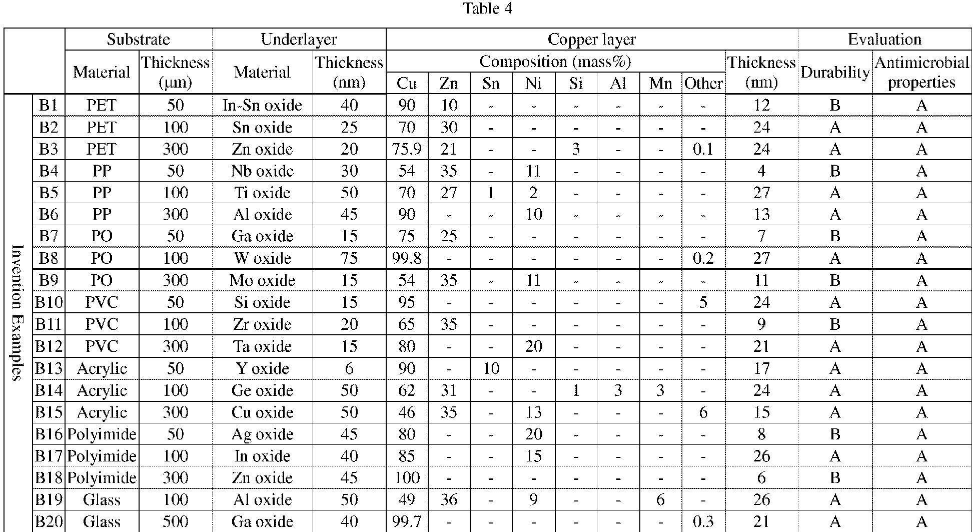

**Table 5**

| | | Substrate | | Underlayer | | Copper layer | | | | | | | | | Evaluation | |
|---|---|---|---|---|---|---|---|---|---|---|---|---|---|---|---|---|
| | | Material | Thickness (µm) | Material | Thickness (nm) | Composition (mass%) | | | | | | | | Thickness (nm) | Bendability | Antimicrobial properties |
| | | | | | | Cu | Zn | Sn | Ni | Si | Al | Mn | Others | | | |
| Invention Examples | B21 | Acrylic | 1000 | Cu oxide | 15 | 85 | 15 | - | - | - | - | - | - | 9 | B | A |
| | B22 | Acrylic | 50 | Nb oxide | 50 | 99 | - | - | - | - | 1 | - | - | 11 | B | A |
| | B23 | Acrylic | 100 | Ti oxide | 40 | 95 | - | 5 | - | - | - | - | - | 11 | A | A |
| | B24 | Acrylic | 500 | In oxide | 9 | 85 | - | - | 15 | - | - | - | - | 7 | B | A |
| | B25 | Acrylic | 100 | Cu oxide | 20 | 90 | 5 | 5 | - | - | - | - | - | 17 | A | A |
| | B26 | Glass | 500 | Sn oxide | 30 | 60 | - | - | 40 | - | - | - | - | 8 | B | A |
| | B27 | Glass | 1000 | In oxide | 40 | 95 | 5 | - | - | - | - | - | - | 15 | A | A |
| | B28 | Glass | 100 | Zn oxide | 50 | 100 | - | - | - | - | - | - | - | 16 | B | A |
| | B29 | Glass | 500 | W oxide | 45 | 54 | 34 | - | 12 | - | - | - | - | 18 | A | A |
| | B30 | Glass | 1000 | Si oxide | 35 | 90 | - | - | 10 | - | - | - | - | 9 | B | A |
| Comparative Example B 1 | | | Glass | - | - | 100 | - | - | - | - | - | - | - | 10 | D | A |

**Table 6**

| | | Transparent substrate | | Underlayer | | Copper layer | | | | | | | | | Surface XPS analysis | |
|---|---|---|---|---|---|---|---|---|---|---|---|---|---|---|---|---|
| | | Material | Thickness (µm) | Material | Thickness (nm) | Composition (mass%) | | | | | | | | Thickness (nm) | CuO/Cu₂O | Antimicrobial properties |
| | | | | | | Cu | Zn | Sn | Ni | Si | Al | Mn | Others | | | |
| Invention Examples | B28 | Glass | 100 | Zn oxide | 50 | 100 | - | - | - | - | - | - | - | 16 | <1 | A |
| | B31 | Glass | 100 | Zn oxide | 50 | 100 | - | - | - | - | - | - | - | 16 | ≥1 | B |

In Comparative Example B1, a copper layer having a thickness of 10 nm was deposited directly on the substrate (glass substrate) without forming an underlayer, and the durability was "D". It is assumed that this was because it was not possible to suppress the generation of aggregation in the copper layer.

In contrast, in Invention Examples B1 to B30, an underlayer formed of a metal oxide was formed between the substrate and the copper layer, the thickness of the copper layer was 30 nm or less, and the durability was excellent.

In addition, in the copper layers having the compositions shown in the table, the bacteria reduction rate was more than 2 in all cases and the evaluation was "A".

Furthermore, as shown in Table 6, in Invention Example B28, the molar ratio CuO/Cu₂O of CuO and Cu₂O in the Cu oxide film formed on the surface layer of the copper layer was less than 1, and it was confirmed that the antimicrobial property of Invention Example B28 was improved in comparison with Invention Example B31 in which the molar ratio CuO/Cu₂O of CuO and Cu₂O in the Cu oxide film was 1 or more.

From the above, according to the Invention Examples, it was confirmed that it is possible to provide a low-cost antimicrobial member with excellent antimicrobial properties and excellent durability.

### [Third Example]

A description will be given below of the results of confirmation experiments performed to confirm the effects of the present invention.

The sputtering target used when depositing the copper layer was manufactured according to the following procedure.

First, as raw materials, a Cu raw material having a purity of 99.9 mass% or higher and metal raw materials having a purity of 99 mass% or higher were weighed to have a predetermined composition. Next, in a melting furnace, the Cu raw material was melted in a high vacuum or in an inert gas atmosphere, and predetermined amounts of the metal raw materials were added to the obtained molten copper. Thereafter, the resultant metal was melted in a vacuum or an inert gas atmosphere to produce a melt-cast ingot. The obtained ingot was subjected to cold rolling, and then a heat treatment was carried out in air, for example, by holding at 600°C for 2 hours and then machining was carried out to produce a disc shape having a diameter of 152.4 mm and a thickness of 6 mm. Thereby, a sputtering target for forming a copper layer was manufactured.

For the sputtering targets used when depositing the underlayer, the sputtering targets listed in Tables 7 and 8, purchased from Kojundo Chemical Laboratory Co., Ltd., were used.

Each of these sputtering targets was joined to a backing plate made of oxygenfree copper by soldering, the resultant target was mounted in a DC magnetron sputtering apparatus, and the inside of the DC magnetron sputtering apparatus was exhausted by a vacuum exhaust system to obtain a degree of vacuum of 5 × 10⁻⁵ Pa or less, then, Ar gas was introduced, and plasma was generated between the cleaned transparent substrates shown in Tables 7 and 8, which were arranged in parallel to the target, and the target described above. Thereby, the underlayer and the copper layer were formed. The deposition conditions for the underlayer and the deposition conditions for the copper layer are as shown below.

### <Deposition Conditions of Underlayer>

Gas used: Ar + 2 volume% of oxygen
Gas pressure: 0.67 Pa
Sputtering power: DC 300 W
Distance between target and substrate: 70 mm

### <Deposition Conditions of Copper Layer>

Peak degree of vacuum: 5×10⁻⁵ Pa or less
Gas used: Ar
Gas pressure: 0.67 Pa
Sputtering power: DC 200W
Distance between target and substrate: 70 mm

The transparent antimicrobial member obtained as described above was evaluated for the following items.

### (Component Composition of Sputtering Target for Forming Copper Layer)

Samples for analysis were taken from the sputtering target for forming the copper layer and the components were measured by ICP emission spectroscopy. It was confirmed that the composition of the sputtering target for forming the copper layer was the same as the composition of the deposited copper layer.

As the compositions of "Others" in the table, it is possible to use Pb, Mg, Zr, Te, Cr, Fe, Co, and the like.

### (Measurement of Film Thickness)

The thicknesses of the underlayer and the copper layer were confirmed by observing the cross-sections of the underlayer and the copper layer with a transmission electron microscope (TEM) and it was confirmed that the layers were deposited to have the targeted thicknesses. For preparing samples for TEM observation, for example, it is possible to use a cross-section polisher (CP) or a focused ion beam (FIB).

### (Transmittance)

The transmittance of the transparent antimicrobial member in the laminating direction was measured using a spectrophotometer (U-4100 manufactured by Hitachi High-Technologies Corporation). The table shows the average transmittance value in a wavelength range from 530 nm to 550 nm. During the measurement, since the baseline was measured using a glass substrate, the values listed in the table are the relative transmittances when the transmittance of the glass substrate is set as 100.

### (Durability Test)

The transparent antimicrobial member was held in an environment at a temperature of 60°C and 90% humidity for 250 hours as a constant temperature and humidity test and the change in color of the surface appearance after the test was confirmed. The change in color was visually determined. A case where the discolored area covered the entire surface of a sample having a size of 40 mm × 40 mm was evaluated as "D", a case where the discolored area was half or more of the entire surface was evaluated as "C", a case where the discolored area was half or less of the entire surface was evaluated as "B", and a case where the discolored area was not seen was evaluated as "A".

### (Evaluation of Antimicrobial Activity)

The antimicrobial activity of the copper and copper alloys of the sputtering targets which were to be deposited on the surface was evaluated as the antimicrobial activity.

The surface of the target used for sputtering was polished with a sandpaper #1000 and a new surface was exposed to make a sample. Thereafter, the surface of each specimen was inoculated with a predetermined amount of Staphylococcus aureus in accordance with JIS Z 2801 and the viable bacteria count (cfu) was quantified after 2 hours. The number of samples was 3, and using the quantified viable bacteria count, the reduction rate of the obtained bacteria was calculated from the following equation. A case where the bacteria reduction rate was more than 2 was evaluated as "A", and a case where the bacteria reduction rate was not more than 2 was evaluated as "C".

Bacteria reduction rate = -log (viable bacteria count (cfu) of specimen after 2 hours/initial inoculation count (cfu))

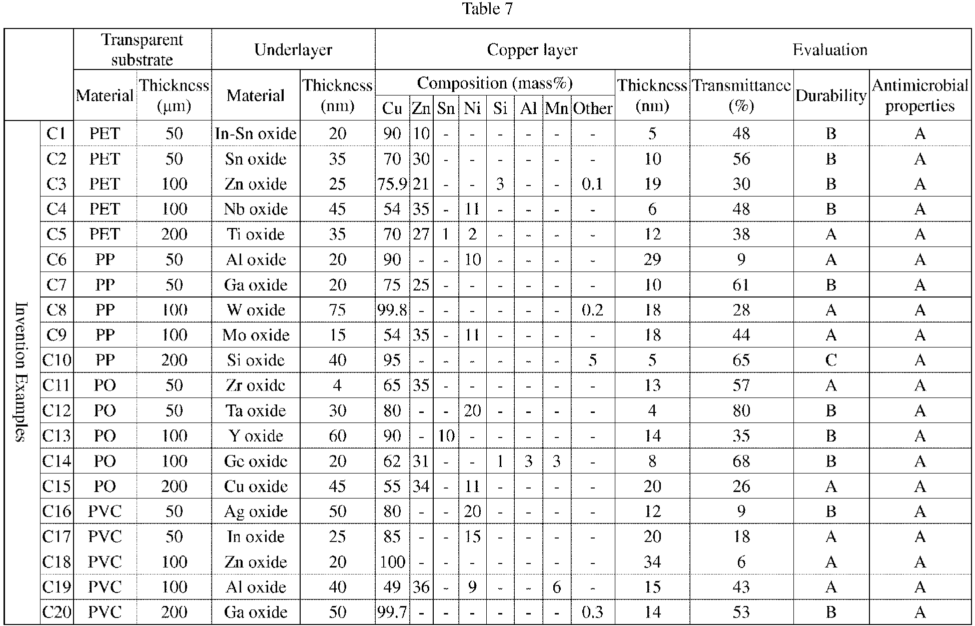

**Table 8**

| | | Transparent substrate | | Underlayer | | Copper layer | | | | | | | | | Evaluation | | |
|---|---|---|---|---|---|---|---|---|---|---|---|---|---|---|---|---|---|
| | | Material | Thickness (µm) | Material | Thickness (nm) | Composition (mass%) | | | | | | | | Thickness (nm) | Transmittance (%) | Durability | Antimicrobial properties |
| | | | | | | Cu | Zn | Sn | Ni | Si | Al | Mn | Others | | | | |
| Invention Examples | C21 | Acrylic | 50 | Cu oxide | 25 | 85 | 15 | - | - | - | - | - | - | 9 | 48 | B | A |
| | C22 | Acrylic | 50 | Nb oxide | 15 | 99 | - | - | - | - | 1 | - | - | 11 | 45 | B | A |
| | C23 | Acrylic | 100 | Ti oxide | 5 | 95 | - | 5 | - | - | - | - | - | 11 | 40 | A | A |
| | C24 | Acrylic | 100 | In oxide | 50 | 85 | - | - | 15 | - | - | - | - | 7 | 47 | B | A |
| | C25 | Acrylic | 200 | Cu oxide | 25 | 90 | 5 | 5 | - | - | - | - | - | 17 | 32 | A | A |
| | C26 | Glass | 50 | Sn oxide | 50 | 60 | - | - | 40 | - | - | - | - | 8 | 60 | B | A |
| | C27 | Glass | 50 | In oxide | 20 | 95 | 5 | - | - | - | - | - | - | 15 | 38 | A | A |
| | C28 | Glass | 100 | Zn oxide | 25 | 100 | - | - | - | - | - | - | - | 16 | 46 | B | A |
| | C29 | Glass | 100 | W oxide | 15 | 54 | 34 | - | 12 | - | - | - | - | 18 | 33 | A | A |
| | C30 | Glass | 200 | Si oxide | 30 | 90 | - | - | 10 | - | - | - | - | 9 | 57 | B | A |
| Comparative Examples | C1 | Glass | 50 | - | - | 100 | - | - | - | - | - | - | - | 10 | 63 | D | A |
| | C2 | PET | 200 | In oxide | 20 | 100 | - | - | - | - | - | - | - | 100 | 0 | A | A |

In Comparative Example C1, a copper layer having a thickness of 10 nm was deposited directly on a transparent substrate (glass substrate) without forming an underlayer, and the durability was "D". It is assumed that this was because it was not possible to suppress the generation of aggregation in the copper layer.

In Comparative Example C2, an underlayer of metal oxide was formed between the transparent substrate (PET substrate) and the copper layer, the copper layer having a thickness of 100 nm was deposited, and the transmittance was 0%. It is assumed that this was because the thickness of the copper layer was excessively thick.

In contrast, in Invention Examples C1 to C30, an underlayer formed of a metal oxide through which visible light was transmitted was formed between the transparent substrate and the copper layer, the thickness of the copper layer was 20 nm or less, and the transmittance was sufficiently high. In addition, the durability was also excellent.

In addition, in the copper layers having the compositions shown in the table, the bacteria reduction rate was more than 2 in all cases and the evaluation was "A".

From the above, it was confirmed that, according to Invention Examples, it is possible to provide a transparent antimicrobial member with excellent antimicrobial properties and transmittance of visible light, which is able to suppress the generation of appearance defects and which is able to be used stably.

### Industrial Applicability

It is possible to provide an antimicrobial member with excellent antimicrobial properties and bendability and which is able to be used stably.

### Explanation of Reference Signs

110: Antimicrobial member
111: Substrate
112: Underlayer
113: Copper layer
115: Adhesive layer
120: Bending test jig
120a: Holding element
120b: Base portion
210: Antimicrobial member
211: Substrate
212: Underlayer
213: Copper layer
215: Adhesive layer
310: Transparent antimicrobial member
311: Transparent substrate
312: Underlayer
313: Copper layer
315: Adhesive layer

## Claims

1. An antimicrobial member comprising:
a substrate;
an underlayer deposited on the substrate; and
a copper layer formed on a surface of the underlayer which is on a side opposite to the substrate and arranged as an outermost layer,
wherein the copper layer is formed of copper or a copper alloy, the underlayer is formed of a metal oxide, and
the substrate is formed of a flexible resin material or a flexible glass material.

2. The antimicrobial member according to claim 1,
wherein a thickness of the copper layer is 5 µm or less.

3. The antimicrobial member according to claim 1 or 2,
wherein a thickness of the copper layer is 35 nm or less.

4. The antimicrobial member according to claim 1,
wherein the substrate is a transparent substrate, and
the metal oxide is a metal oxide through which visible light is transmitted.

5. The antimicrobial member according to any one of claims 1 to 4,
wherein no cracking is confirmed after carrying out a bending test 100 times under a condition where a radius of curvature is 6 mm.

6. The antimicrobial member according to any one of claims 1 to 5,
wherein a thickness of the underlayer is 500 nm or less.

7. The antimicrobial member according to any one of claims 1 to 6,
wherein a thickness of the underlayer is 100 nm or less.

8. The antimicrobial member according to any one of claims 1 to 7,
wherein a thickness of the underlayer is 50 nm or less.

9. The antimicrobial member according to any one of claims 1 to 8,
wherein a thickness of the substrate is 500 µm or less.

10. The antimicrobial member according to any one of claims 1 to 9,
wherein the metal oxide that forms the underlayer includes any one or more selected from In oxide, Sn oxide, Zn oxide, Nb oxide, Ti oxide, Al oxide, Ga oxide, W oxide, Mo oxide, Si oxide, Zr oxide, Ta oxide, Y oxide, Ge oxide, Cu oxide, and Ag oxide.

11. The antimicrobial member according to any one of claims 1 to 10,
wherein the copper layer is formed of a copper alloy which includes one or more selected from the group consisting of Zn, Sn, Ni, Al, Si, and Mn at a total amount of 0.1 mass% or more and in which an amount of Cu is 45 mass% or more.

12. The antimicrobial member according to any one of claims 1 to 11,
wherein a Cu oxide film is formed on a surface layer of the copper layer, and a molar ratio CuO/Cu₂O of CuO and Cu₂O in the Cu oxide film is CuO/Cu₂O < 1.

13. The antimicrobial member according to any one of claims 1 to 12,
wherein an adhesive layer is provided on a surface of the substrate on a side opposite to the underlayer.

14. The antimicrobial member according to any one of claims 1 to 13,
wherein a transmittance with respect to light having a wavelength of 550 nm in a laminating direction is 5% or more.
